# EUROPEAN PATENT SPECIFICATION

(11) **EP 2 616 010 B1**
(45) Date of publication and mention of the grant of the patent: **12.04.2017**
(21) Application number: 11737971.9
(22) Date of filing: 01.08.2011
(51) Int. Cl.: A61F 2/36

(54) **MODULAR PROSTHETIC DEVICE FOR HIP JOINT REPLACEMENT**
MODULARE PROTHETISCHE VORRICHTUNG FÜR EINE HÜFTGELENKSPROTHESE
DISPOSITIF PROSTHÉTIQUE MODULAIRE DESTINÉ À REMPLACER L'ARTICULATION DE LA HANCHE

(30) Priority: 14.09.2010 IT MI20101668
(43) Date of publication of application: 24.07.2013
(73) Proprietor: Adler Ortho S.R.L., 20032 Cormano (IT); Schiraldi, Marco, 12038 Savigliano (IT); Renzi Brivio, Lodovico, 37131 Verona (IT)
(72) Inventor: SCHIRALDI, Marco, 12038 Savigliano (IT); RENZI BRIVIO, Lodovico, I-37131 Verona (IT); CREMASCOLI, Patrizio, I-20091 Bresso (IT)
(74) Representative: Modiano, Micaela Nadia
(86) International application number: PCT/EP2011/063195
(87) International publication number: WO 2012/034771

(56) References cited:
- FR-A1- 2 700 947
- FR-A1- 2 767 471
- US-A1- 2003 074 079
- US-B1- 6 464 728

## Description

### Technical Field

The present invention relates to a modular prosthetic device for hip joint replacement and to a device for determining the level of resection of the neck of the femur in order to optimize the implantation of said modular prosthetic device.

### Background art

Currently, the treatment of degenerative hip arthrosis by means of artificial prostheses is very successful.

The hip prosthesis is an artificial joint made of metal alloys, plastic materials and/or ceramics, and constituted by a cup and a stem, which are inserted respectively in the acetabulum and in the femur. A spherical prosthetic head made of metal or ceramic is assembled onto the stem and is articulated to the inner surface of the cup.

As is known, the femur is a long bone in which a body and two ends can be distinguished. The upper end has a head and two raised portions known as trochanters. The head has a spherical shape so as to be articulated to the acetabulum and is supported by a bone segment known as a neck, at the base of which the two trochanters are located. In particular, the greater trochanter is located laterally and upward and the lesser trochanter is located centrally and further down.

During hip prosthesis implantation, most of the neck and the head of the femur are usually removed, since they are replaced by the artificial components of the prosthesis. Then, after appropriate preparation of the medullar canal of the femur, the stem of the prosthesis is inserted within the canal thus created.

In the current background art, the stems of known hip prostheses are particularly long, usually 130-190 mm. The implantation of long femoral stems makes it necessary to provide a particularly invasive excavation of the femoral canal and does not allow the possibility of sparing much of the available bone; moreover, the implantation of these femoral stems does not allow the provision of small incisions at the surface level and the respect of soft tissues.

In view of the drawbacks affecting these particularly long known stems, attempts have been made to provide stems that have smaller dimensions. However, reducing the size of the stem causes the inevitable reduction of the contact surface between the bone and the prosthesis, thus highlighting increasingly the need for the surfaces of the stem that make contact with the bone to have high osteointegration.

A femoral implant prosthesis, having the features set forth in the preamble of claim 1, is known from US 6 464 728 B1.

### Disclosure of the invention

The aim of the present invention is to provide a modular prosthetic device that makes it possible to overcome the limitations of the background art.

Within this aim, an object of the invention is to provide a modular prosthetic device that allows the respect of soft tissues, minimal invasion of the femoral canal, sparing of available bone and reconstruction of muscle tension so that the possibility to perform any subsequent implantations is not prevented.

Another object of the invention is to provide a modular prosthetic device whose implantation is performed by means of an implant that has the least possible invasiveness, i.e., in which surgical access to the hip joint occurs by performing mini-incisions.

Another object of the present invention is to provide a modular prosthetic device which, in addition to eliminating the pain caused by the degenerative disease, provides advantages from a functional standpoint, such as those related to the size of the wound and to restoration of the exact length of the limb and of muscle tension.

A further object of the present invention is to provide a modular prosthetic device that allows good primary fixation, i.e., fixation obtained during prosthesis implantation, which ensures stability of said prosthesis within the bone, primary fixation being therefore a fixation of the mechanical type, and good secondary fixation, i.e., fixation related to biological reconstruction.

Another object of the present invention is to provide a modular prosthetic device that ensures rapid reeducation and restoration of motor functions linked to the hip joint.

A further object of the invention is to provide a modular prosthetic device that is highly reliable, relatively easy to provide and has competitive costs.

This aim and these and other objects which will become better apparent hereinafter are achieved by a modular prosthetic device for hip joint replacement, according to the present invention, that has the features set forth in claim 1.

### Brief description of the drawings

Further characteristics and advantages of the invention will become better apparent from the description of a preferred but not exclusive embodiment of the modular prosthetic device according to the invention, illustrated by way of non-limiting example in the accompanying drawings, wherein:

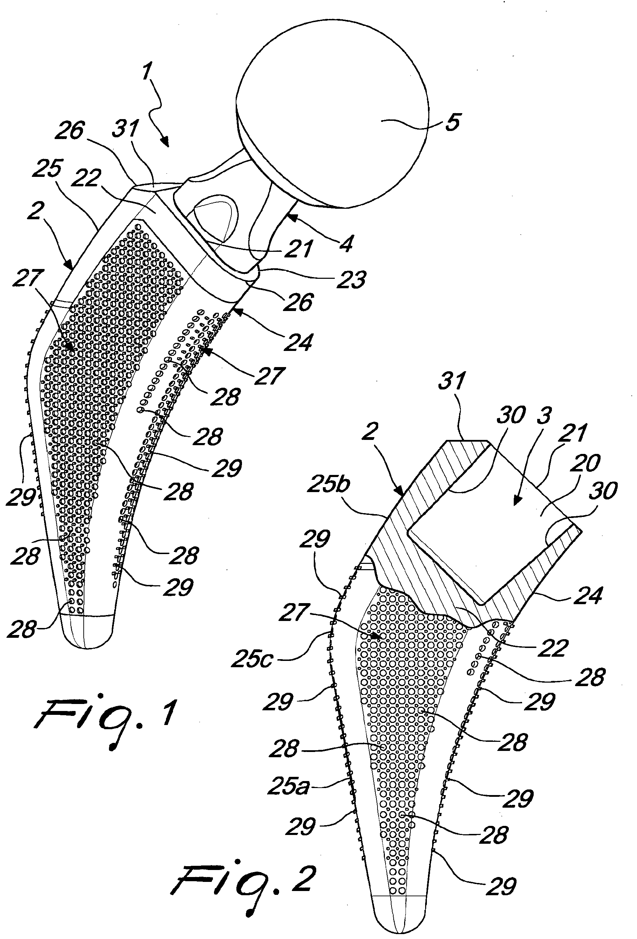

Figure 1 is a perspective view of a modular prosthetic device according to the invention;
Figure 2 is a sectional front view of the upper part of a stem of the modular prosthetic device according to the invention;
Figure 3 is an exploded perspective view of the modular prosthetic device according to the invention;
Figure 4 is a perspective view of a femur with which a first embodiment of a device for determining the level of resection of the neck of the femur is associated;
Figure 5 is a perspective view of a femur with which a second embodiment of a device for determining the level of resection of the neck of the femur is associated.

### Ways of carrying out the invention

With reference to the figures, the modular prosthetic device for hip joint replacement according to the invention, generally designated by the reference numeral 1, comprises a stem 2, which is inserted within the femoral canal shaped by the surgeon.

According to the invention, the stem 2 comprises in an upward region a cavity 20 which defines a receptacle 3 for accommodating the lower portion 40 of a neck element 4.

The lower portion 40 of the neck element 4 is inserted within the cavity 20, forming a stable and safe mating.

More precisely, insertion occurs by pushing the neck element 4 into the cavity 20; a lower end face 41 of the neck element 4 and a transverse cross-section 21 of the cavity 20 have substantially identical shapes and dimensions, thus allowing, as a consequence of said insertion, relative contact between first walls 45 of the neck element 4 and second walls 30 of the cavity 20. This contact creates such a friction force as to ensure a stable and safe mating.

An upper portion 42 of the neck element 4 is inserted within a spherical element 5, which defines the head of the prosthetic device 1 that is designed to be articulated within the acetabular cup (not shown in the accompanying drawings).

More precisely, the stem 2 is defined by four faces: a front face 22, a rear face 23, a central face 24 and a lateral face 25.

The front face 22 and the rear face 23 are substantially identical and are mutually arranged so as to be substantially parallel. More precisely, when the device 1 is inserted in the femur, the front face 22 and the rear face 23, being mutually parallel, are both parallel to a front plane.

The mutually facing ends of the front face 21 and of the rear face 23 are connected by the central face 24 and by the lateral face 25. More precisely, the central face 24 has a substantially arc-like longitudinal extension, which is curved in a regular and constant manner; the lateral face 25 instead has a longitudinal extension that comprises a lower portion 25a and an upper portion 25b, which are straight or slightly inclined and are connected centrally by a curved region 25c.

The upper portion of the central face 24 and of the lateral face 25 are mutually substantially symmetrical on the sagittal plane with respect to the individual into whom the device 1 is implanted.

The lateral face 25 is provided, in an upper region, with a region 31 that is inclined downwardly with respect to the plane of the transverse cross-section 21 of the cavity 20.

The central face 24 and the lateral face 25 have a substantially semielliptical, preferably semicircular transverse profile 26. The fact that the semielliptical transverse profile 26 extends along the entire longitudinal path of the central face 24 and the lateral face 25 makes it possible to avoid excessive stresses on the neck of the femur and to minimize the risks of fracture.

Advantageously, the front face 22, the rear face 23, the central face 24 and the lateral face 25 have a porous surface 27.

More precisely, the porous surface 27 comprises a plurality of circular recesses 28 and a plurality of cylindrical protrusions 29, both of which are arranged in an orderly fashion on the surface 27.

The porous surface 27 is intended to facilitate osteointegration of the device 1 with the tissues of the femoral canal in which the stem 2 is implanted.

As already mentioned, the neck element 4 comprises a lower portion 40, which is inserted in the cavity 20 defined in the stem 2, and an upper portion 42, which is inserted at the plane that intersects the spherical element 5.

More precisely, the lower portion 40 comprises two hemispherical bodies 43 and 43a, which extend starting from the lower end face 41, whose shape and dimensions are substantially equal to those of the transverse cross-section 21 d and 21c of the cavity 20. More particularly, the lower end face 41 comprises two first sides 44a and 44b, which are concave and mutually identical and are joined, at their respective ends, to substantially semicircular contours 44c and 44d.

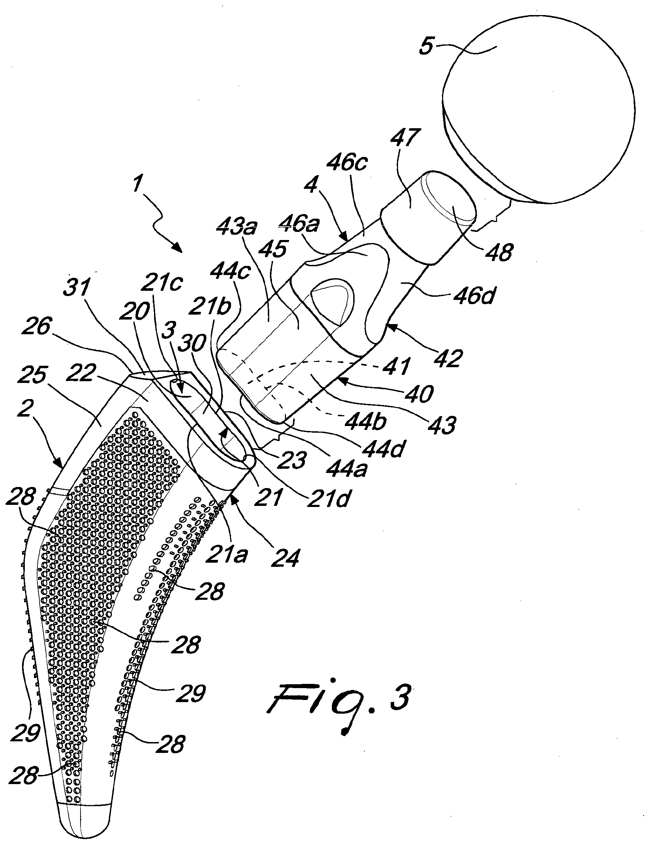

In a substantially similar manner, the cavity 20 comprises two first sides 21a and 21b, which are convex and mutually identical and which are joined, at their respective ends, to second substantially semicircular contours 21c and 21d (see Figure 3).

Advantageously, the bodies 43 and 43a that rise from the lower end face 41 expand gradually in a transverse direction so that they are slightly inclined outwardly; likewise, the cavity 20 that extends downwardly from the transverse cross-section 21 can taper gradually in a transverse direction, so that the walls 21d and 21c of the cavity 20 are slightly inclined inwardly. The mutual inclination of the walls of the body 40 and of the walls of the cavity 20 makes it possible to increase mutual contact between said walls, thus facilitating a stable and effective mating.

The upper portion 42 of the neck element 4 is defined by four sectors 46a, 46b, 46c and 46d, which are identical and symmetrical in pairs. The four sectors 46a and 46c and 46b and 46d have rounded contours and move away from the lower portion, defining a longitudinal profile. In particular, as the sectors 46 move away from the lower portion 40, they move closer together until they are associated in an upward region with a tubular element 47 that has a circular upper end face 48. The tubular element 47 and the upper end face 48 of the neck element 4 are inserted within the opening defined on the flat face of the spherical element 5.

Both the stem 2 and the neck element 4 are made of highly biocompatible materials, such as titanium alloys. Preferably, the stem 2 is provided starting from powders of the titanium alloy Ti6A14V.

Advantageously, the stem 2 can be provided entirely by means of a technique for melting by an electron beam Ti6A14V of titanium alloy powders (better known as EBM, "Electron Beam Melting").

As an alternative, the stem 2 is provided by laser sintering of Ti6A14V titanium alloy powders.

More precisely, in the prosthetic device 1 the anterior/posterior thicknesses vary, among the different sizes, substantially by a value comprised between 0.1 mm and 0.2 mm.

Moreover, in the prosthetic device 1, the lateral-central dimension between the largest stem 2 and the smallest stem 2 varies in the proximal region by approximately 3 mm and in the distal region by approximately 11 mm.

Moreover, the stem 2 of the prosthetic device 1 has distal lateral-central dimensions that vary from 9 mm to 20 mm depending on the size; with such dimensions, the stems do not touch the cortical regions of the femur.

In practice it has been found that the device according to the invention fully achieves the intended aim, since by providing separately and in a modular manner the neck element and the stem it is possible to separate the choice of the stem from the choice of the neck element, so that for each implant it is possible to select independently the best suited stem and neck element.

For each implant in each patient it is possible to select the neck element and the stem that make it possible to optimize bone sparing, muscle tension, limb length, offset and any anterior/posterior version.

More precisely, the fact that in the prosthetic device according to the invention the neck element and the stem are not provided as a single component but as two distinct components that can be mated stably allows the easy provision of prosthetic devices of different sizes and configurations simply by mating stems and neck elements having different shapes and sizes. Moreover, by doing so in practice the number of available combinations is increased; a given size of the stem can in fact mate with all sizes of the neck element.

More particularly, the different sizes of the stems vary in terms of the size and shape of the four faces (anterior, posterior, central and lateral), while the cavity inside which the lower portion of the neck element is inserted is identical for all sizes.

Likewise, the neck elements of the different sizes can have, according to the requirements, a different upper portion, both in terms of geometry and in terms of length and inclination; while the lower portion, which is inserted in the cavity of the stem, is the same for all sizes.

Thanks to the use of the modular necks and by operating on the resection of the neck of the femur (while at the same time always respecting the principle of sparing the bone of the neck as much as possible), in critical cases it is possible to adapt the length of the saved bone to the requirements of press-fit.

Parva stems have, with respect to traditional stems, even those that use modular necks, an additional adjustment possibility, which is the length of the resection of the neck of the femur.

If press-fit with the lateral cortex of the femur requires a size that is not long enough to use all the spared bone, it is possible to perform a resection of part of the spared bone and use a longer neck.

Although the modular prosthetic device 1 according to the invention has been conceived in particular for the hip joint, it can in any case be used more generally for other joints in which there is a stem and a neck element.

Some known devices, as well as the modular prosthetic device 1 according to the invention, make it possible to preserve the neck of the femur 201, i.e., they avoid complete surgical removal of the neck of the femur 201 prior to implantation.

However, currently commercially available devices that preserve the neck of the femur 201 tend to alter the length of the limb.

In particular, in case of implantation of the modular prosthetic device 1, in order to spare more bone of the neck of the femur 201 without increasing the length of the limb, the surgeon must perform a resection of the neck of the femur 201 at such a level that the shorter neck element 4 allows the center of the spherical element 5 mounted on the neck element 4 to coincide with the center 203 of the head of the femur 202 of the patient prior to the operation.

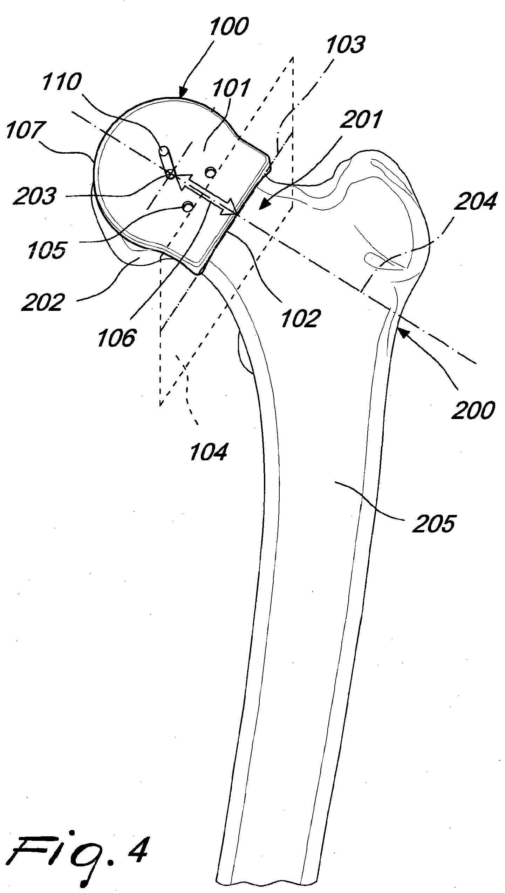

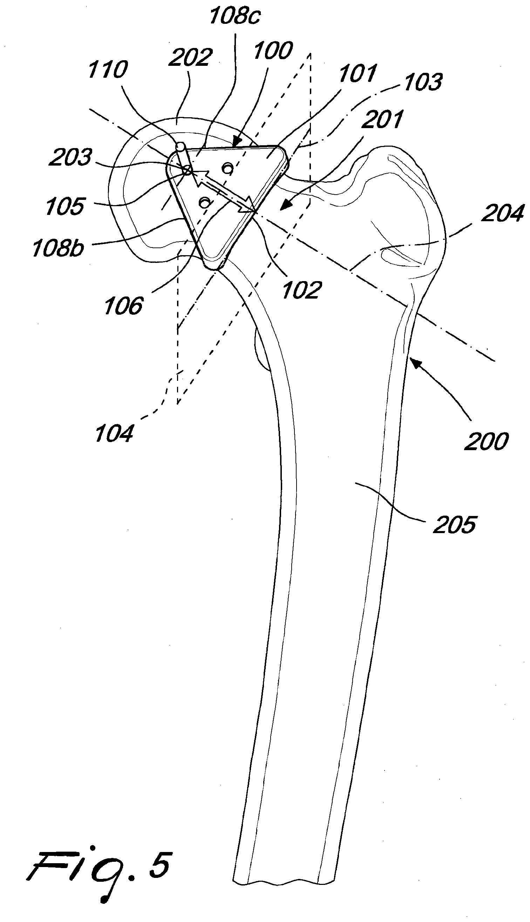

For facilitating this operation, according to the invention a device 100 for performing resection of the neck of the femur 201, as shown in Figures 4 and 5, is provided.

The device 100 comprises a plate 101, which can be associated with the center of rotation 203 of the head of the femur 202. The plate 101 has at least one straight side 102 for defining a resection line 103 that is substantially perpendicular to the longitudinal direction 204 of the neck of the femur 201.

The resection line 103 defines a plane 104 for resection of the neck of the femur 201 that is substantially perpendicular to the plane of extension of the plate 101 and to the longitudinal direction 204 of the neck of the femur 201.

Advantageously, the plate 101 comprises centrally one or more holes 105 whose shape and dimensions are such as to allow the passage through them of a reference pivot 110, which is fixed on the center of rotation 203 of the head of the femur 202.

Advantageously, the hole 106 and the reference pivot 110 can be mutually shaped so that the respective interlocking coupling automatically ensures correct alignment of the straight side 102 with respect to the longitudinal direction 204 of the neck of the femur 201.

The distance 106 between the hole 105, crossed by the reference pivot 110, and the straight side 102 defines the portion of the neck of the femur 201 that is removed.

In a first embodiment, shown in Figure 4, the plate 101 has a curved side 107 which is C-shaped, the ends of the C-shape being connected to the ends of the straight side 102. In this first embodiment, the plate 101 covers completely the head of the femur 202.

As an alternative, the plate 101 can have a shape that does not cover completely the head of the femur 52. By way of non-limiting example, the plate 101 of Figure 5 has two catheti 108c and 108b which are arranged so as to form a triangle with the straight side 102.

In both embodiments, the ends of the straight side 102 or of any other sides 108c and 108b are substantially bevelled so as to reduce the risk of damage, including accidental damage, of the tissues that surround the head of the femur.

Operation of the device 100 according to the invention for performing resection of the neck of the femur is as follows.

First of all, the surgeon determines, by means of known instruments and methods, the center 203 of the head of the femur 202 and inserts the reference pivot 110 therein.

The surgeon then locks the device 100 on the femur 200, fitting the hole 105 of the plate 101 of the device 100 on the reference pivot 110.

If the hole 106 and the reference pivot 110 are not shaped complementary to allow automatically the correct alignment of the straight side 102 with respect to the longitudinal direction 204 of the neck of the femur 201, the surgeon can adjust manually the orientation of the plate 101 so as to achieve said correct alignment.

At this point, the surgeon performs resection along the resection plane 103 defined by the straight side 102 and substantially at right angles to the longitudinal direction 204 of the neck of the femur 201.

Moreover, the surgeon, during the preparation of the femur body 205 inside which the stem 2 is to be inserted, ensures that the proximal portion of the excavation provided inside the femur body 205 reaches the height at which the resection of the neck of the femur 201 has been performed.

In practice it has been found that the device according to the invention fully achieves the intended aim, since it makes it possible to define a resection plane along which resection of the neck of the femur is performed.

Although the device 100 according to the invention for determining the resection of the neck of the femur has been conceived in particular for resection of the neck of the femur, it can in any case be used more generally for the resection of bones of other joints (shoulder, elbow, etcetera).

The device thus conceived is susceptible of numerous modifications and variations, all of which are within the scope of the appended claims; all the details may further be replaced with other technically equivalent elements.

In practice, the materials used, as well as the dimensions, may be any according to requirements and to the state of the art.

Reference is made to Italian Patent Application No. MI2010A001668 from which this application claims priority.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A modular prosthetic device (1) for hip joint replacement, comprising a stem (2) adapted to be inserted within the femur, a modular neck element (4) that comprises an upper portion (42) having in an upper region a tubular element (47) and a lower portion (40), and a spherical head (5), said modular neck element (4) being coupleable with its tubular element (47) provided in its upper region to said spherical head (5), and wherein said stem (2) and modular neck element (4) are constituted by two distinct components that can be stably mated upon selection thereof among a plurality of stems (2) and neck elements (4) having different shapes and sizes so as to provide prosthetic devices of different sizes and configurations,
said stem (2) comprising, in an upper region, a cavity (20) for the safe accommodation of the lower portion (40) of said neck element (4),
**characterized in that** said lower portion (40) of said neck elements (4) comprises two bodies (43, 43a) of substantially semicircular contours (44c, 44d) which extend starting from a lower end face (41) of said lower portion (401 and whose shape and dimensions are substantially equal to those of a transverse cross-section (21d, 21c) of said cavity (20),
and **in that** said lower end face (41) of said lower portion (40) comprises two first sides (44a, 44b) which are concave and mutually identical and are joined, at their respective ends, to said substantially semicircular contours (44c,44d) of said bodies, (43, 43a), said cavity (20) comprising two first sides (21a, 21b) which are convex and mutually identical and which are joined, at their respective ends, to second substantially semicircular contours (21c, 21d) of said cavity (20).

2. The device according to claim 1, **characterized in that** the transverse cross-section of said cavity (20) and the lower end face of said lower portion (40) of said neck element (4) have substantially identical shapes and dimensions.

3. The device according to one or more of the preceding claims, **characterized in that** said neck element (4) is pushed within said cavity (20) of said stem (2) and is locked by the friction force that is created as a consequence of contact between first walls of said neck element (4) and second walls of said cavity (20).

4. The device according to one or more of the preceding claims, **characterized in that** said stem (2) comprises a front face (22), a rear face (23), a central face (24) and a lateral face (25); said front face (22) and said rear face (23) being substantially identical and mutually parallel; the mutually facing ends of said front face (22) and of said rear face (23) being connected by said central face (24) and by said lateral face (25).

5. The device according to one or more of the preceding claims, **characterized in that** said central face (24) and said lateral face (25) have a substantially semicircular transverse profile.

6. The device according to one or more of the preceding claims, **characterized in that** at least one of said front face (22), said rear face (23), said central face (24) and said lateral face (25) has a porous surface in order for facilitating osteointegration.

7. The device according to one or more of the preceding claims, **characterized in that** said stem (2) has distal dimensions comprised substantially between 8 mm and 22 mm so as to avoid contact with the cortical regions of the femur.

8. The device according to one or more of the preceding claims, **characterized in that** said stem (2) has distal dimensions comprised substantially between 9 mm and 20 mm so as to avoid contact with the cortical regions of the femur.

## Patentansprüche

1. Eine modulare prothetische Vorrichtung (1) für eine Hüftgelenksprothese, die Folgendes umfasst: einen Stamm (2), ausgebildet, um in den Oberschenkel eingesetzt zu werden, ein modulares Schaftelement (4), das einen oberen Abschnitt (42) umfasst, der in einem oberen Bereich ein röhrenförmiges Element (47) und einen unteren Abschnitt (40) und einen Kugelkopf (5) hat, wobei das modulare Schaftelement (4) über sein röhrenförmiges Element (47), das in seinem oberen Bereich angebracht ist, mit dem Kugelkopf (5) koppelbar ist und wobei der Stamm (2) und das modulare Schaftelement (4) aus zwei separaten Komponenten bestehen, die nach Auswahl derselben aus einer Vielzahl von Stämmen (2) und Schaftelementen (4) mit verschiedenen Formen und Größen stabil verbunden werden können, um prothetische Vorrichtungen mit verschiedenen Größen und Anordnungen zu bilden,
wobei der Stamm (2) in einem oberen Bereich einen Hohlraum (20) für die sichere Aufnahme des unteren Abschnitts (40) des Schaftelement (4) umfasst,
**dadurch gekennzeichnet, dass** der untere Abschnitt (40) des Schaftelements (4) zwei. Körper (43, 43a) mit im Wesentlichen halbkreisförmigen Konturen (99c, 44d) umfasst, die sich ausgehend von einer unteren Endfläche (41) des unteren Abschnitts (40) erstrecken und deren Form und Maße im Wesentlichen gleich denjenigen eines transversalen Querschnitts (21d, 21c) des Hohlraums (20) sind,
und dadurch, dass die untere Endfläche (41) des unteren Abschnitts (40) zwei erste Seiten (44a, 44b) umfasst, die konkav und miteinander identisch und an ihren jeweiligen Enden mit den im Wesentlichen halbkreisförmigen Konturen (49c, 44d) der Körper (43, 43a) verbunden sind, wobei der Hohlraum (20) zwei erste Seiten (21a, 21b) umfasst, die konvex und miteinander identisch und an ihren jeweiligen Enden mit zweiten im Wesentlichen halbkreisförmigen Konturen (21c, 21d) des Hohlraums (20) verbunden sind.

2. Die Vorrichtung gemäß Anspruch 1, **dadurch gekennzeichnet, dass** der transversale Querschnitt des Hohlraums (20) und die untere Endfläche des unteren Abschnitts (40) des Schaftelements (4) im Wesentlichen identische Formen und Maße haben.

3. Die Vorrichtung gemäß einem oder mehreren der obigen Ansprüche, **dadurch gekennzeichnet, dass** das Schaftelement (4) in den Hohlraum (20) des Stamms (2) geschoben und durch die Reibungskraft blockiert wird, die infolge des Kontakts zwischen ersten Wänden des Schaftelements (4) und zweiten Wänden des Hohlraums (20) erzeugt wird.

4. Die Vorrichtung gemäß einem oder mehreren der obigen Ansprüche, **dadurch gekennzeichnet, dass** der Stamm (2) eine vordere Fläche (22), eine hintere Fläche (23), eine zentrale Fläche (24) und eine Seitenfläche (25) umfasst; wobei die vordere Fläche (22) und die hintere Fläche (23) im Wesentlichen identisch und zueinander parallel sind; die einander gegenüberliegenden Enden der vorderen Fläche (22) und der hinteren Fläche (23) über die zentrale Fläche (24) und die Seitenfläche (25) verbunden sind.

5. Die Vorrichtung gemäß einem oder mehreren der obigen Ansprüche, **dadurch gekennzeichnet, dass** die zentrale Fläche (24) und die Seitenfläche (25) ein im Wesentlichen halbkreisförmiges Querprofil haben.

6. Die Vorrichtung gemäß einem oder mehreren der obigen Ansprüche, **dadurch gekennzeichnet, dass** mindestens eine der vorderen Fläche (22), der hinteren Fläche (23), der zentralen Fläche (24) und der Seitenfläche (25) eine poröse Oberfläche hat, um die Osteointegration zu erleichtern.

7. Die Vorrichtung gemäß einem oder mehreren der obigen Ansprüche, **dadurch gekennzeichnet, dass** der Stamm (2) distale Maße hat, die im Wesentlichen zwischen 8 mm und 22 mm betragen, um Kontakt mit den kortikalen Bereichen des Oberschenkels zu vermeiden.

8. Die Vorrichtung gemäß einem oder mehreren der obigen Ansprüche, **dadurch gekennzeichnet, dass** der Stamm (2) distale Maße hat, die im Wesentlichen zwischen 9 mm und 20 mm betragen, um Kontakt mit den kortikalen Bereichen des Oberschenkels zu vermeiden.

## Revendications

1. Dispositif prothétique modulaire (1) pour le remplacement d'une articulation de hanche, comprenant une tige (2) adaptée pour être insérée à intérieur du fémur, un élément de col modulaire (4) qui comprend une partie supérieure (42) ayant, dans une région supérieure, un élément tubulaire (47) et une partie inférieure (40), et une tête sphérique (5), ledit élément de col modulaire (4) pouvant être couplé, avec son élément tubulaire (47) prévu dans sa région supérieure, à ladite tête sphérique (5), et dans lequel ladite tige (2) et l'élément de col modulaire (4) sont constitués par deux composants distincts qui peuvent être couplés de manière stable suite à leur sélection, parmi une pluralité de tiges (2) et d'éléments de col (4) ayant différentes formes et tailles afin de fournir des dispositifs prothétiques de différentes tailles et configurations,
ladite tige (2) comprenant, dans une région supérieure, une cavité (20) pour loger en toute sécurité la partie inférieure (40) dudit élément de col (4),
**caractérisé en ce que** ladite partie inférieure (40) dudit élément de col (4) comprend deux corps (43, 43a) de contours sensiblement semi-circulaires (44c, 44d) qui s'étendent à partir d'une face d'extrémité inférieure (41) de ladite partie inférieure (40) et dont la forme et les dimensions sont sensiblement égales à celles d'une section transversale (21d, 21c) de ladite cavité (20),
et **en ce que** ladite face d'extrémité inférieure (41) de ladite partie inférieure (40) comprend deux premiers côtés (44a, 44b) qui sont concaves et mutuellement identiques et sont assemblés, au niveau de leurs extrémités respectives, auxdits contours sensiblement semi-circulaires (44c, 44d) desdits corps (43, 43a), ladite cavité (20) comprenant deux premiers côtés (21a, 21b) qui sont convexes et mutuellement identiques et qui sont assemblés au niveau de leurs extrémités respectives, aux seconds contours sensiblement semi-circulaires (21c, 21d) de ladite cavité (20).

2. Dispositif selon la revendication 1, **caractérisé en ce que** la section transversale de ladite cavité (20) et la face d'extrémité inférieure de ladite partie inférieure (40) dudit élément de col (4) ont des formes et des dimensions sensiblement identiques.

3. Dispositif selon une ou plusieurs des revendications précédentes, **caractérisé en ce que** ledit élément de col (4) est poussé à l'intérieur de ladite cavité (20) de ladite tige (2) et est bloqué par la force de friction qui est créée en conséquence d'un contact entre des premières parois dudit élément de col (4) et des secondes parois de ladite cavité (20).

4. Dispositif selon une ou plusieurs des revendications précédentes, **caractérisé en ce que** ladite tige (2) comprend une face avant (22), une face arrière (23), une face centrale (24) et une face latérale (25) ; ladite face avant (22) et ladite face arrière (23) étant sensiblement identiques et mutuellement parallèles ; les extrémités se faisant mutuellement face de ladite face avant (22) et de ladite face arrière (23) étant raccordées par ladite face centrale (24) et par ladite face latérale (25).

5. Dispositif selon une ou plusieurs des revendications précédentes, **caractérisé en ce que** ladite face centrale (24) et ladite face latérale (25) ont un profil transversal sensiblement semi-circulaire.

6. Dispositif selon une ou plusieurs des revendications précédentes, **caractérisé en ce qu'**au moins l'une parmi ladite face avant (22), ladite face arrière (23), ladite face centrale (24) et ladite face latérale (25) a une surface poreuse afin de faciliter l'ostéointégration.

7. Dispositif selon une ou plusieurs des revendications précédentes, **caractérisé en ce que** ladite tige (2) a des dimensions distales sensiblement comprise entre 8 mm et 22 mm afin d'éviter le contact avec les régions corticales du fémur.

8. Dispositif selon une ou plusieurs des revendications précédentes, **caractérisé en ce que** ladite tige (2) a des dimensions distales sensiblement comprise entre 9 mm et 20 mm afin d'éviter le contact avec les régions corticales du fémur.
